Europäisches Patentamt

European Patent Office

Office européen des brevets

(11) Numéro de publication : **0 169 151**
**B1**

(12) **FASCICULE DE BREVET EUROPÉEN**

(45) Date de publication du fascicule du brevet :
27.07.88

(21) Numéro de dépôt : **85420096.1**

(22) Date de dépôt : **21.05.85**

(51) Int. Cl.⁴ : **A 61 M 16/00**, A 61 M 15/00

(54) **Appareil portable pour inhalation d'air chaud et humidifié.**

(30) Priorité : **22.05.84 FR 8408190**

(43) Date de publication de la demande :
**22.01.86 Bulletin 86/04**

(45) Mention de la délivrance du brevet :
**27.07.88 Bulletin 88/30**

(84) Etats contractants désignés :
**AT CH IT LI SE**

(56) Documents cités :
**FR-A- 2 162 340**
**GB-A- 526 678**
**US-A- 1 771 366**
**US-A- 2 091 034**

(73) Titulaire : **CENTRE NATIONAL DE LA RECHERCHE SCIENTIFIQUE (CNRS)**
**15, Quai Anatole France**
**F-75007 Paris (FR)**

(72) Inventeur : **Dittmar, André LA. 181 - C.N.R.S.**
**Faculté de Médecine 8, Avenue Rockfeller**
**F-69373 Lyon Cedex 08 (FR)**
Inventeur : **Foray, Jacques**
**Hôpital**
**F-74403 Chamonix Mont Blanc Cedex (FR)**
Inventeur : **Delhomme, Georges**
**57, rue Saint Jérôme**
**F-69007 Lyon (FR)**
Inventeur : **Blain, Yves**
**"Le Victoria" Vaulnaveys Le Haut"**
**F-38400 Uriage (FR)**
Inventeur : **Berard, Pierre Service Technique Tunnel Routier Sous Le Mont Blanc**
**F-74400 Chamonix Mont Blanc (FR)**

(74) Mandataire : **Ropital-Bonvarlet, Claude et al**
**Cabinet BEAU DE LOMENIE 99, Grande rue de la Guillotière**
**F-69007 Lyon (FR)**

EP 0 169 151 B1

Jouve, 18, rue St-Denis, 75001 Paris, France

## Description

La présente invention concerne les dispositifs ou appareils utilisés pour lutter contre l'hypothermie chez l'homme par inhalation d'air chaud et elle vise, à titre principal, les dispositifs pouvant être qualifiés de portables ou d'autonomes, c'est-à-dire ceux destinés à être utilisés sur les lieux mêmes de l'accident.

Pour lutter contre l'hypothermie, les expérimentations ont permis de mettre en évidence l'intérêt de l'apport de chaleur à l'accidenté par la voie pulmonaire.

En effet, le corps humain peut être considéré comme divisible en deux parties du point de vue de sa thermo-régulation. La partie la plus sensible ou noyau thermique est celle dont la température est normalement réglée à 37° C. Cette partie du corps comprend le cerveau, le cœur, le foie, etc... La seconde partie est celle pouvant être considérée comme périphérique à la première et pour laquelle des températures variables peuvent être acceptées, compte tenu des fonctions organiques à maintenir et des capacités d'échanges avec le milieu environnant. Cette seconde partie comprend, essentiellement, les muscles et les membres.

Une différence notable de température dans ces deux parties du corps humain peut donc être acceptée, à condition toutefois que celle du noyau thermique ne descende pas en dessous de 30° C. En effet, en dessous de cette température, le cœur fonctionne mal et risque de s'arrêter.

Des expériences ont permis de constater que l'apport de chaleur doit être, obligatoirement, assuré au noyau thermique du corps humain, de façon à pouvoir maintenir les organes vitaux considérés dans des conditions de fonctionnement optimal, permettant de lutter contre une hypothermie localisée au territoire périphérique ou à la seconde partie.

On a constaté que l'apport d'air chaud, par l'intermédiaire des poumons directement, permettait d'atteindre ce but, à condition que cet air chaud soit, par ailleurs, suffisamment chargé en humidité, afin d'éviter le risque des spasmes bronchiques et de déssèchement des voies respiratoires.

Pour assurer l'apport d'un air chaud et humide, en vue de lutter contre l'hypothermie, plusieurs propositions ont été formulées.

L'une d'elles consiste à chauffer, par un système à combustion extérieure du type réchaud, un récipient contenant de l'eau, de façon à produire une vapeur d'eau chaude qui est inhalée par le sujet.

Un tel procédé est dangereux, car la vapeur d'eau ne peut être contrôlée ou régulée en température et risque alors, dans certains cas, d'être responsable de brûlures des voies respiratoires.

Par ailleurs, le fonctionnement d'un tel dispositif exige une position stable horizontale qui ne peut pas être pratiquement adoptée dans les faits, étant donné qu'un risque d'hypothermie est, la plupart du temps, la conséquence d'un accident dans un lieu ou site difficile d'accès ou encombré.

En outre, l'utilisation d'un réchaud, généralement à réserve de gaz combustible, représente une source de danger et d'accidents non négligeable.

Une seconde proposition s'appuie sur la réaction exothermique de la chaux mise en présence d'eau. Une telle réaction exothermique ne permet pas de contrôler ou réguler la température du gaz de réaction de façon précise et ne permet donc pas de répondre au problème posé qui implique, obligatoirement, la fourniture d'un mélange ou d'un air humidifié à température régulée constante pour placer le sujet dans un état de réceptivité, de décontraction et d'attente les plus favorables à la lutte contre l'hypothermie qu'il subit accidentellement.

On conçoit, par ailleurs, qu'une telle proposition est difficile à mettre en œuvre pratiquement dans le cas d'accidents de la route ou de sauvetages en montagne. Par ailleurs, une telle technique ne permet pas de disposer d'une autonomie de fonctionnement suffisante pour la lutte pratique contre l'hypothermie.

Une troisième proposition peut être considérée sur la base de l'enseignement divulgué par le document FR-A-2 162 340 bien qu'il ne concerne pas, à proprement parlé, la lutte contre l'hypothermie chez l'homme, mais vise le traitement médical des voies respiratoires.

Selon ce document, le dispositif de traitement préconisé comprend un carter tubulaire isolant définissant une entrée et une sortie d'air, un circuit de circulation interne à rebroussements successifs et contenant une unité de chauffage disposée dans la dernière partie du circuit et un moyen d'humidification placé en amont de l'unité de chauffage. Des moyens de régulation de la température d'air sont également prévus.

Un tel dispositif n'apparaît pas adapté au domaine visé par l'invention car, entre autres, il se range parmi les dispositifs à caractère statique fonctionnant par dépression résultant de l'aspiration par le sujet. En outre, le montage en série du moyen d'humidification et de l'unité de chauffage n'apparaît pas approprié à la fourniture d'un air convenablement chauffé, humidifié et contrôlé pour l'application à la lutte contre l'hypothermie de l'homme.

La présente invention vise à remédier aux inconvénients attachés aux propositions actuelles connues de lutte contre l'hypothermie par inhalation d'air chaud humide et, à cette fin, a pour objet un appareil portable possédant une grande autonomie de fonctionnement et pouvant être utilisé dans toutes les positions.

L'objet de l'invention est de proposer un appareil permettant de fonctionner efficacement dans une plage de température importante et sous une pression atmosphérique variable, pour délivrer un air chaud et humide régulé à une température maximale réglable, optimale pour la fonction de réchauffement du noyau thermique et sans aucun risque pour les voies respiratoires.

Un autre objet de l'invention est de proposer un appareil facilement manipulable par un personnel non spécialement qualifié et présentant une résistance, notamment aux chocs, très élevée.

Un autre objet de l'invention est de proposer un appareil de faible encombrement, de manière à pouvoir être introduit, hissé, engagé ou placé dans tout endroit par lequel un corps humain est déjà passé, afin de rendre possible toutes les opérations de sauvetage d'un être humain placé dans des conditions d'hypothermie à la suite d'un accident.

L'objet de l'invention est particulièrement choisi dans sa conception, de façon à représenter un matériel de sauvetage dans les accidents de montage ou les accidents de la circulation routière ou auto-routière ou encore maritime entre autres.

Pour atteindre les buts ci-dessus, l'objet de l'invention est caractérisé :

— en ce qu'il comprend :

un tuyau flexible en communication avec l'orifice de sortie du carter qui canalise l'air vers un orifice buccal et qui comporte des moyens de décharge et une sonde de mesure de la température de l'air,

un dispositif de mise en circulation de l'air selon une direction axiale au carter et logé dans un compartiment de ce dernier communiquant avec l'orifice d'entrée,

— en ce que l'unité de chauffage et d'humidification de l'air est située entre le compartiment et l'orifice de sortie, de sorte que la gaine d'écoulement d'air s'étende entre le compartiment et l'orifice de sortie,

— et en ce que les moyens de régulation contrôlent l'alimentation du faisceau de résistances électriques et comprenant la sonde de mesure de la température de l'air et deux thermostats associés à l'unité.

Diverses autres caractéristiques ressortent de la description faite ci-dessous en référence aux dessins annexés qui montrent, à titre d'exemples non limitatifs, des formes de réalisation de l'objet de l'invention.

La fig. 1 est une coupe-élévation schématique de l'objet de l'invention.

La fig. 2 est une coupe transversale prise selon la ligne II-II de la fig. 1.

La fig. 3 est un schéma fonctionnel de l'un des éléments constitutifs de l'objet de l'invention.

La fig. 4 est une coupe transversale analogue à la fig. 2, mais illustrant une variante de réalisation.

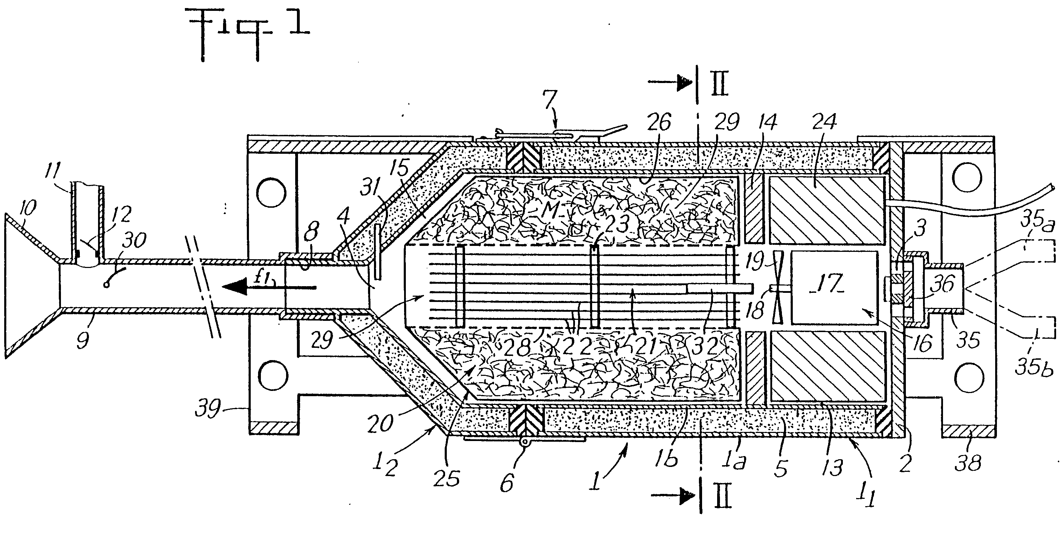

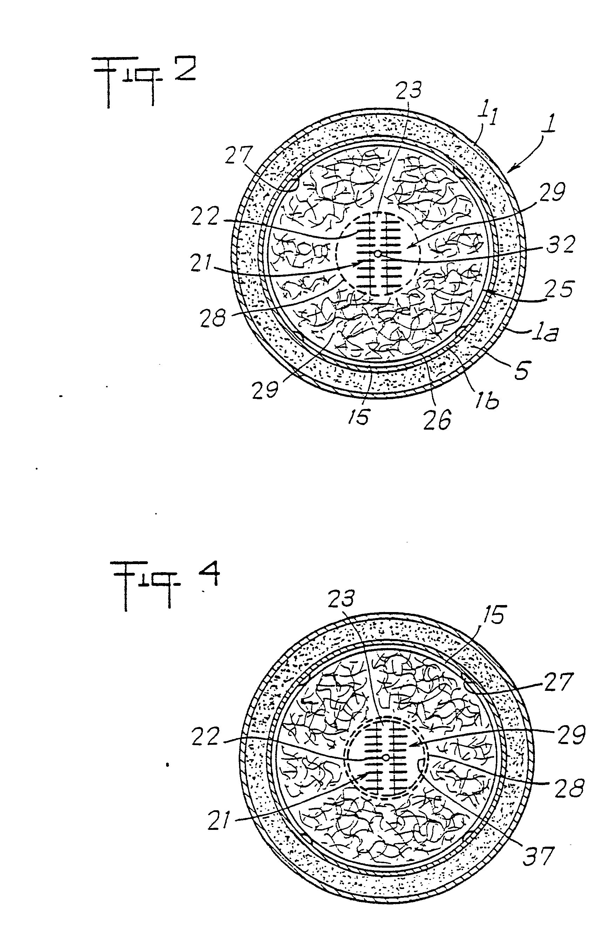

Selon les fig. 1 et 2, l'appareil comprend un carter ou boîtier 1, de forme générale tubulaire, fermé à une extrémité transversale par une paroi 2 délimitant un orifice d'entrée d'air 3. L'extrémité opposée du carter ou boîtier 1 présente un orifice 4 de sortie d'air, qui est placé sensiblement sur l'axe longitudinal géométrique du carter ou boîtier 1.

Dans une forme de réalisation, le carter est constitué par deux enveloppes concentriques 1a et 1b, en toute matière ayant de bonnes caractéristiques de résistance mécanique et d'isolation thermique. Ces deux enveloppes 1a et 1b sont réunies entre elles par un garnissage ou un remplissage 5 en matière isolante, par exemple en une matière développée en mousse, à cellules ouvertes ou fermées, dont la densité est choisie de manière qu'elle contribue à l'obtention de caractéristiques de résistance mécanique élevée, en étant intimement liée aux faces en regard des enveloppes concentriques 1a et 1b.

Selon une construction préférée, le boîtier ou carter 1 comprend un corps principal $1_1$ et un corps secondaire $1_2$ réalisé sous la forme d'un couvercle. Le corps secondaire $1_2$ est monté sur le corps $1_1$ par une articulation 6 et par au moins un dispositif de fixation 7 du type sauterelle par exemple.

Le corps secondaire $1_2$ délimite l'orifice de sortie 4 qui est bordé par un raccord 8 sur lequel s'adapte, de façon permanente ou temporaire, un tuyau 9 pourvu, à son extrémité libre, d'un embout buccal ou d'un masque 10. Le tuyau 9 comporte, immédiatement en amont de l'embout 10, une dérivation ou une mise à l'air libre 11 dont la section de passage est contrôlée par un clapet de décharge 12 sensible à la pression régnant dans le tuyau 9.

Le corps principal $1_1$ délimite, à partir de la paroi transversale 2, un compartiment 13 séparé par une cloison transversale partielle 14 d'un second compartiment 15 délimité, en partie par le corps principal $1_1$ et en partie par le corps secondaire $1_2$.

Le compartiment 13 contient un dispositif 16 de mise en circulation d'air prélevé extérieurement au carter ou boîtier 1 par l'orifice d'entrée 3. Le dispositif 16 peut être constitué par un moteur électrique 17 fixé, de toute manière appropriée, directement ou indirectement sur la cloison transversale 2. Le moteur 17 comporte un arbre de sortie 18 s'étendant dans l'axe du corps 1 et portant une hélice 19 chargée de propulser de l'air ou un mélange gazeux, dans le sens de la flèche $f_1$, à travers la cloison 14 en direction du compartiment 15. Le moteur électrique 17 est alimenté en courant électrique, de préférence, à partir d'une source de distribution indépendante à laquelle l'appareil de l'invention est raccordé par un cordon d'alimentation.

Dans certains cas, il peut être prévu de conférer au compartiment 13 un volume suffisant pour y loger une source de production autonome, telle qu'une batterie rechargeable.

Le compartiment 15 contient une unité 20 de chauffage et d'humidification. L'unité 20 comprend un faisceau 21 de résistances électriques 22, de préférence du type lamellaire, qui sont maintenues parallèlement les unes aux autres par des barrettes 23, tel qu'illustré par les fig. 1 et 2. Le faisceau 21 est prévu pour s'étendre coaxialement à l'axe du carter 1 sur au moins une partie de la longueur du compartiment 15. Le faisceau 21 est raccordé électriquement à des moyens de régulation 24 chargés de contrôler l'alimentation des résistances à partir de la source de production ou de distribution d'énergie électrique. Les moyens 24 sont, de préférence, réalisés sous une forme annulaire, de façon à pouvoir être disposés dans le compartiment 13 en entourant le dispositif 16 de mise en circulation de l'air.

L'unité 20 comprend, par ailleurs, une cartouche d'humidification 25 annulaire comportant une paroi extérieure 26, de forme tubulaire, dont le diamètre extérieur est prévu pour permettre un engagement juste dans le compartiment 15. A cet effet, la paroi périphérique interne du compartiment 15 peut comporter des nervures axiales 27 de centrage et d'immobilisation. La cartouche 25 comporte, en outre, une paroi interne 28 concentrique à la paroi 26 et délimitant un évidement central 29 représentant, à la fois, une gaine de circulation pour l'air propulsé par le dispositif 16 et un logement pour le faisceau 21. La paroi périphérique interne 28 est du type ajouré et peut être constituée par un grillage, un métal déployé, une feuille ou plaque perforée, etc...

Les parois périphériques 26 et 28 sont réunies entre elles par des parois transversales définissant un logement annulaire rempli d'une masse M de matière fibreuse ou analogue, ayant un caractère hydrophile marqué. A titre d'exemple, la matière M peut être constituée par de la cellulose en fibre formée en mèches.

La cartouche 25 est conformée, de façon complémentaire au volume du compartiment 15, de façon à pouvoir être immobilisée axialement dans ce dernier entre la cloison 14 et la face interne du corps secondaire $1_2$, lesquelles peuvent comporter, bien que cela ne soit pas représenté, des bossages de butée ou, de préférence, des tampons rapportés en matière déformable élastiquement.

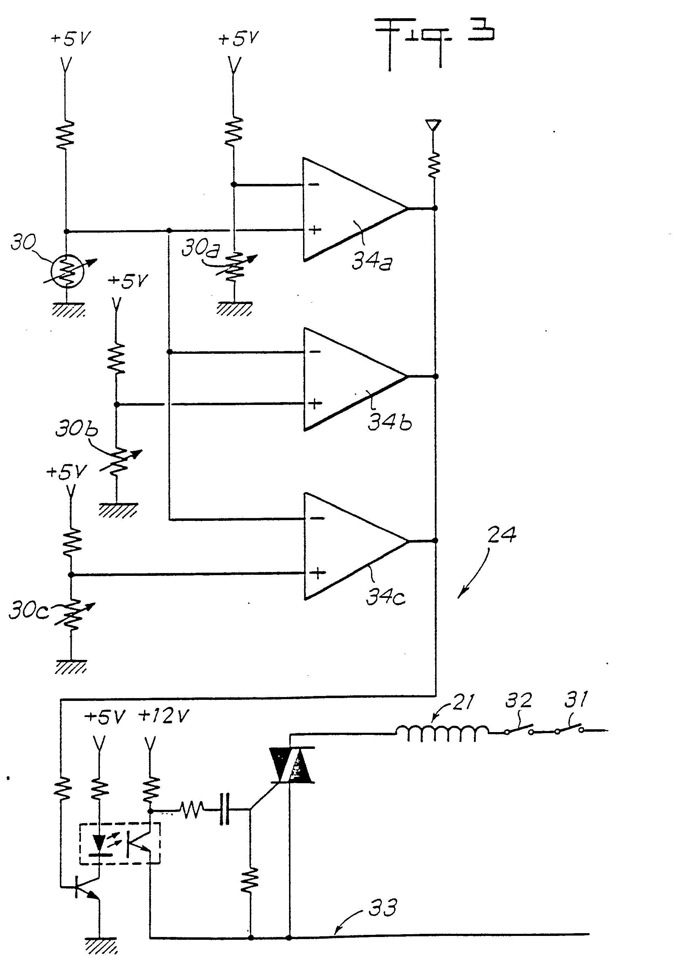

Les moyens de régulation 24, dont un schéma illustratif est donné à titre d'exemple par la fig. 3, comprennent une sonde 30 de mesure de la température de l'air. Selon l'invention, cette sonde 30 est disposée au centre du tuyau 9 à proximité de l'embout 10 et, dans tous les cas, légèrement en amont de la dérivation ou mise à l'air libre 11. Les moyens de régulation 24 comprennent également un premier thermostat 31 disposé dans le compartiment 15 pour apprécier la température de l'air issu de l'unité 20 et empruntant le tuyau 9. Les moyens 24 comprennent, par ailleurs, un second thermostat 32 appréciant la température développée par le faisceau 21 dans le logement 29.

Les thermostats 31 et 32 sont disposés en série sur le circuit de puissance 33 d'alimentation du faisceau 21 contrôlé par les moyens de régulation 24. Selon la fig. 3, ces moyens 24 comprennent de plus des potentiomètres réglables 30a, 30b et 30c associés à des comparateurs 34a, 34b et 34c représentant des éléments de sécurité chargés de maintenir l'intégrité de la liaison électrique entre la sonde 30 et les moyens d'ouverture-fermeture du circuit 33.

Il doit être considéré que les moyens de régulation 24 pourraient être réalisés de toute autre façon différente à la portée de l'homme de l'art.

L'appareil décrit ci-dessus fonctionne de la façon suivante.

Lors de la mise sous tension, le dispositif 16 propulse, selon la flèche $f_1$, de l'air prélevé du milieu ambiant à travers le compartiment 15. La mise sous tension ferme également le circuit 33, de sorte que les résistances 22 du faisceau 21 sont alimentées en énergie électrique. L'air propulsé par le dispositif 16 traverse le faisceau au sein duquel il s'échauffe avant d'emprunter le tuyau 9.

L'élévation de température du faisceau 21 provoque l'évaporation de l'eau retenue par la matière M de la cartouche 25. Cette évaporation s'effectue à travers la paroi périphérique ajourée 28 dans le logement 29, de sorte que l'air chauffé est simultanément chargé en vapeur d'eau, avant d'emprunter le tuyau 9.

L'air chauffé et humidifié est maintenu à température constante par l'intermédiaire du capteur 30 agissant sur les moyens de régulation 24. Ainsi, le sujet en hypothermie peut inhaler, par l'embout 10, un air à température constante régulé en fonction de l'apport calorifique devant être effectué, des conditions d'hypothermie et des réglages apportés, notamment par le potentiomètre 30a.

Le maintien d'une température constante, pour l'air empruntant le tuyau 9, est assuré par le capteur 30, quelles que soient les déperditions thermiques pouvant intervenir en fonction des conditions d'utilisation le long du trajet du tuyau 9. Le débit d'air chauffé et humidifié, excédentaire par rapport à la consommation du sujet en traitement, est refoulé par la dérivation 11 qui peut assumer une fonction de simple mise à l'air libre ou une fonction d'acheminement de cette fraction excédentaire pour réchauffer la zone périphérique du corps ou certains des membres du sujet.

Pendant le fonctionnement de l'appareil, l'évaporation des couches de matière M, les plus proches du faisceau 21, entretient une migration radiale vers l'intérieur de l'eau retenue dans les couches périphériques. Il en résulte une perte progressive permettant d'entretenir une évaporation à un taux sensiblement constant et de produire ainsi un air chaud humidifié sans variation importante de son hygrométrie.

La disposition de la cartouche 25, autour du faisceau 21, permet de faire assumer à la masse M une fonction supplémentaire d'isolation thermique permettant de réduire les déperditions radiales en combinaison avec le caractère isolant du carter 1.

Il importe de noter que le dispositif 16 et les moyens de régulation 24 sont disposés entre l'orifice d'entrée 3 et l'unité 20, de telle sorte que ces organes ne sont pas soumis à une montée en température lors du fonctionnement de l'appareil, ni à des contraintes thermiques susceptibles d'altérer leur fonctionnement, notamment dans le temps.

L'orifice 3 est, de préférence, muni d'un embout 35 associé à un obturateur 36 de réglage de sa section de passage. L'embout 35 permet, soit l'aspiration directe à partir du milieu ambiant, soit le raccordement à une source fournissant un mélange gazeux approprié au traitement devant être conduit.

La fig. 1 montre que l'embout 35 peut comporter, aussi, deux tubulures 35a et 35b permettant, le cas échéant, de prélever de l'air du milieu ambiant et, en même temps, un mélange gazeux complémentaire fourni par un réservoir d'appoint.

La fig. 4 montre, par comparaison avec la fig. 2, que la paroi périphérique interne 28 de la cartouche 25 peut être associée à un fourreau 37 mobile axialement ou angulairement. Un tel fourreau représente un organe de réglage de la surface d'échange entre la cartouche et le logement 29. Il devient possible, par ce fourreau, de régler initialement le taux d'humidification de l'air chaud refoulé et, le cas échéant, d'agir de cette façon sur l'autonomie de fonctionnement de l'appareil.

Dans ce qui précède, il est indiqué que le carter 1 est construit en deux parties, afin de faciliter le montage et le changement de la cartouche 25. Il peut aussi être envisagé de munir le carter 1 d'un orifice de remplissage permettant de recharger la cartouche par un apport d'eau extérieur.

La fig. 2 fait apparaître que le corps principal $1_1$ et le corps secondaire $1_2$ sont prolongés vers l'extérieur par des anneaux de préhension 38 et 39 constituant, par ailleurs, des organes pare-chocs protégeant les embouts de raccordement 35 et 38.

Ainsi que cela ressort de ce qui précède, les moyens mis en œuvre pour constituer l'objet de l'invention permettent d'obtenir un appareil compact, portable et capable de délivrer un air chaud à température régulée et humidifié de façon sensiblement constante.

La disposition structurelle retenue selon l'invention permet, par ailleurs, de disposer d'un appareil susceptible de fonctionner dans toutes les positions, en étant partiellement ou totalement autonome, selon que l'énergie électrique nécessaire à son fonctionnement est prélevée à partir d'une source de distribution extérieure ou est fournie par une batterie propre.

Par ailleurs, les conditions de fonctionnement de l'appareil sont indépendantes de la température extérieure et de la pression atmosphérique. En outre, l'isolation thermique, assurée par la constitution du carter et la disposition concentrique de la cartouche 25 par rapport au faisceau 21, permet d'obtenir, dans des conditions extrêmes difficiles, une production d'air chaud à température régulée et humidifiée pendant une durée importante, sans modification de réglage de fonctionnement.

L'invention n'est pas limitée aux exemples décrits et représentés, car diverses modifications peuvent y être apportées sans sortir de son cadre.

**Revendications**

1. Appareil portable de lutte contre l'hypothermie chez l'homme par inhalation d'air chaud et humidifié, du type comprenant :
 — un carter tubulaire (1) isolant, constitué par un corps principal ($1_1$) définissant un orifice d'entrée (3) d'air et par un couvercle ($1_2$) délimitant un orifice de sortie (4) d'air,
 — une unité (20) de chauffage et d'humidification de l'air, disposée dans un compartiment (15) du carter et constituée, d'une part, par une cartouche annulaire amovible (25) de matière hydrophile, délimitant par son évidement central axial (29) une gaine d'écoulement d'air et, d'autre part, par un faisceau (21) de résistances électriques (22) s'étendant à l'intérieur de cette gaine,
 — des moyens de régulation (24) de la température de l'air délivré par l'unité (20), caractérisé :
 — en ce qu'il comprend :
 un tuyau flexible (9) en communication avec l'orifice de sortie (4) du carter (1) qui canalise l'air vers un orifice buccal et qui comporte des moyens de décharge (11, 12) et une sonde (30) de mesure de la température de l'air,
 un dispositif (16) de mise en circulation de l'air selon une direction axiale au carter et logé dans un compartiment (13) de ce dernier communiquant avec l'orifice d'entrée (3),
 — en ce que l'unité (20) de chauffage et d'humidification de l'air est située entre le compartiment (13) et l'orifice de sortie (4), de sorte que la gaine d'écoulement d'air s'étende entre le compartiment (13) et l'orifice de sortie (4),
 — et en ce que les moyens de régulation (24) contrôlent l'alimentation du faisceau (21) de résistances électriques et comprennent la sonde (30) de mesure de la température de l'air et deux thermostats (31, 32) associés à l'unité (20).

2. Appareil selon la revendication 1, caractérisé en ce que le dispositif (16) de mise en circulation et les moyens de régulation (24) sont disposés dans un même compartiment (13) situé entre l'orifice d'entrée d'air (3) et l'unité de chauffage et d'humidification (20).

3. Appareil selon la revendication 2, caractérisé en ce que les moyens de régulation (24) sont réalisés sous la forme d'un corps annulaire entourant le dispositif (16) de mise en circulation de l'air.

4. Appareil selon les revendications 2 ou 3, caractérisé en ce que les moyens de régulation (24) sont associés à un thermostat (31) placé à proximité de l'orifice de sortie (4) et à un second thermostat (32) disposé au sein des résistances de chauffage (22).

5. Appareil selon la revendication 1, caractérisé en ce que les résistances électriques (22) sont de formes lamellaires et s'étendent parallèlement au sens de circulation d'air.

6. Appareil selon la revendication 1, caractérisé en ce que la cartouche (25) comporte une enveloppe périphérique extérieure (26) pleine et une enveloppe périphérique intérieure ajourée (28) délimitant l'évidement central (29) et, entre ces deux enveloppes, une masse (M) de matière hydrophile.

7. Appareil selon la revendication 6, caractérisé en ce que l'unité (20) comprend une cartouche (25) d'humidification dont la paroi périphérique interne ajourée est associée à un fourreau mobile (36) de réglage de la surface de communication entre le volume interne de la cartouche et l'évidement central.

8. Appareil selon la revendication 1, caractérisé en ce que le carter (1) est constitué par deux enveloppes concentriques (1a-1b) délimitant entre elles un intervalle annulaire rempli d'une matière (5) à bon coefficient d'isolation thermique.

9. Appareil selon la revendication 1, caractérisé en ce que l'orifice d'entrée d'air (3) est muni d'un embout de raccordement (35) associé à un obturateur (36) de réglage de sa section de passage.

## Claims

1. Portable device for combatting hypothermia in humans by inhalation of warm, humidified air, of the type comprising :
— an insulating tubular housing (1) constituted by a main body ($1_1$) defining an air intake opening (3) and a cover ($1_2$) defining an air outlet (4),
— an air heating and humidifying unit (20), placed in a compartment (15) of the housing and constituted, on the one hand, by a removable annular cartridge (25) in a hydrophilic material, defining through its axial central recess (29) an air flowing conduit, and on the other hand, by a bundle (21) of electrical elements (22) extending inside said conduit,
— means for controlling (24) the temperature of the air supplied by unit (20), characterized :
— in that it comprises :
a flexible pipe (9) communicating with the outlet (4) of the housing (1) which channels the air towards a buccal orifice and which comprises discharge means (11, 12) and an air temperature measuring probe (30),
an air circulating device (16) following an axial direction of the housing and housed in a compartment (13) thereof, communicating with the admission opening (3),
— in that the air heating and humidifying unit (20) is placed between the compartment (13) and the outlet (4), so that the air flowing conduit extends between the compartment (13) and the outlet (4),
— and in that the control means (24) control the supply of the bundle (21) of electrical elements and comprise the air temperature measuring probe (30) and two thermostats (31, 32) associated with unit (20).

2. Device according to claim 1, characterized in that the circulating device (16) and the control means (24) are placed in the same compartment (13) situated between the air admission opening (3) and the heating and humidifying unit (20).

3. Device according to claim 2, characterized in that the control means (24) are in the form of an annular body surrounding the air circulating device (16).

4. Device according to claims 2 or 3, characterized in that the control means (24) are associated with a thermostat (31) placed close to the air outlet (4) and with a second thermostat (32) placed inside the heating elements (22).

5. Device according to claim 1, characterized in that the electrical elements (22) have a lamellar shape and extend in parallel to the air flowing direction.

6. Device according to claim 1, characterized in that the cartridge (25) comprises a solid external peripheral cover (26) and a perforated internal peripheral cover (28) defining the central recess (29) and between both these covers, a mass (M) of hydrophilic material.

7. Device according to claim 6, characterized in that the unit (20) comprises a humidifying cartridge (25) of which the internal peripheral perforated wall is associated with a movable bush (36) for adjusting the communication surface between the internal volume of the cartridge and the central recess.

8. Device according to claim 1, characterized in that the housing (1) is cons tituted by two concentric covers (1a-1b) defining therebetween an annular gap filled with a material (5) having a good heat insulation coefficient.

9. Device according to claim 1, characterized in that the air admission opening (3) is provided with a connecting socket (35) associated with a shutter (36) for adjusting its passage section.

## Patentansprüche

1. Tragbares Gerät zur Verhinderung einer Unterkühlung beim Menschen durch Inhalieren warmer befeuchteter Luft mit
— einem rohrförmigen isolierenden Gehäuse (1), das aus einem Grundkörper ($1_1$) besteht, der eine Lufteingangsöffnung (3) definiert und mit einem Deckel ($1_2$), der eine Luftausgangsöffnung (4) begrenzt,
— einer Einheit (20) zur Heizung und Befeuchtung von Luft, die in einem Abteil (15) des Gehäuses untergebracht ist und einerseits aus einer auswechselbaren ringförmigen Hülse (25) aus hydrophilem Material, die durch ihre mittlere axiale Aussparung (29) einen Luftkanal bildet und die andererseits aus einem Bündel (21) elektrischer Widerstände (22) besteht, die sich im Inneren dieser Hülsen erstrecken, und
— Organen (24) zur Regelung der Temperatur der durch die Einheit (20) gelieferten Luft, gekennzeichnet durch
— ein flexibles Rohr (9), das in Verbindung mit der Ausgangsöffnung (4) des Gehäuses (1) steht und die Luft zu einer bukkalen Öffnung hin kanalisiert und das Auslaßorgane (11, 12) und eine Sonde (30) zur Messung der Lufttemperatur aufweist,
— eine Vorrichtung (16) zur Bewegung der Luft in einer axialen Richtung im Verhältnis zum Gehäuse, die in einem Abteil (13) dieses Gehäuses untergebracht ist, das mit der Eingangsöffnung (3) in Verbindung steht, wobei die Einheit (20) zur Heizung und Befeuchtung von Luft zwischen dem Abteil (13) und der Ausgangsöffnung (4) angeordnet ist, derart, daß der Luftströmungskanal sich zwischen dem Abteil (13) und der Ausgangsöffnung (4) erstreckt, und

— die Regelorgane (24) die Speisung des elektrischen Widerstandsbündels (21) regeln und eine Lufttemperaturmeßsonde (30) und zwei mit der Einheit (20) verbundene Thermostate (31, 32) aufweisen.

2. Gerät nach Anspruch 1, dadurch gekennzeichnet, daß die Vorrichtung (16) zum Bewegen der Luft und die Regelorgane (24) in demselben Abteil (13) angeordnet sind, das zwischen der Lufteingangsöffnung (3) und der Einheit zur Heizung und Befeuchtung (20) angeordnet ist.

3. Gerät nach Anspruch 2, dadurch gekennzeichnet, daß die Regelorgane (24) in Form von Ringkörpern ausgebildet sind, die die Vorrichtung (16) zur Bewegung der Luft umschließen.

4. Gerät nach Anspruch 2 oder 3, dadurch gekennzeichnet, daß die Regelorgane (24) mit einem Thermostaten (31) verbunden sind, der in der Nähe der Ausgangsöffnung (4) angeordnet ist und mit einem zweiten Thermostaten (32), der in unmittelbarer Nähe der Heizwiderstände (22) angeordnet ist.

5. Gerät nach Anspruch 1, dadurch gekennzeichnet, daß die elektrischen Widerstände (22) eine Lamellenform haben und sich parallel zur Luftströmungsrichtung erstrecken.

6. Gerät nach Anspruch 1, dadurch gekennzeichnet, daß die Hülse (25) einen äußeren geschlossenen Umfangsmantel (26) und einen inneren gelochten Umfangsmantel (28) aufweist, der die zentrale Ausnehmung (29) umgrenzt und dadurch, daß die Hülse zwischen den beiden Mänteln eine Masse (M) aus einem hydrophilen Material aufweist.

7. Gerät nach Anspruch 6, dadurch gekennzeichnet, daß die Einheit (20) eine Befeuchtungshülse (25) aufweist, deren innere gelochte Mantelfläche eine bewegliche Hülse (36) zum Einstellen der Verbindungsfläche zwischen dem Innenvolumen der Patrone und der zentralen Ausnehmung aufweist.

8. Gerät nach Anspruch 1, dadurch gekennzeichnet, daß das Gehäuse (1) aus zwei konzentrischen Hüllen (1a, 1b) besteht, die zwischen sich einen Ringraum einschließen, der mit einem Material (5) gefüllt ist, das einen guten thermischen Isolationskoeffizienten hat.

9. Gerät nach Anspruch 1, dadurch gekennzeichnet, daß die Lufteingangsöffnung (3) mit einem Verbindungsansatz (35) versehen ist, der mit einem Schließorgan (36) verbunden ist, zur Einstellung seiner Durchlaßöffnung.

Fig. 1

# Fig- 2

# Fig- 4

Fig 3